# EUROPÄISCHE PATENTSCHRIFT

(11) **EP 0 901 456 B1**
(45) Veröffentlichungstag und Bekanntmachung des Hinweises auf die Patenterteilung: **27.12.2000**
(21) Anmeldenummer: 97922922.6
(22) Anmeldetag: 30.04.1997
(51) Int. Cl.: C07C 67/317, C07C 69/74

(54) **VERFAHREN ZUR HERSTELLUNG VON 2-FLUOR-1-CYCLOPROPANCARBONSÄUREDERIVATEN DURCH REDUKTIVE ENTHALOGENIERUNG**
PROCESS FOR PRODUCING 2-FLUORINE-1-CYCLOPROPANE CARBOXYLIC ACID DERIVATIVES BY REDUCTIVE DEHALOGENATION
PROCEDE POUR FABRIQUER DES DERIVES DE L'ACIDE 2-FLUORO-1-CYCLOPROPANE CARBOXYLIQUE PAR DESHALOGENATION REDUCTRICE

(30) Priorität: 23.05.1996 DE 19620798
(43) Veröffentlichungstag der Anmeldung: 17.03.1999
(73) Patentinhaber: BAYER AG, 51368 Leverkusen (DE)
(72) Erfinder: WOLTERS, Erich, D-50939 Köln (DE); LUI, Norbert, D-51060 Köln (DE); MÜLLER, Nikolaus, D-40789 Monheim (DE)
(86) Internationale Anmeldenummer: EP9702210
(87) Internationale Veröffentlichungsnummer: WO9744309

(56) Entgegenhaltungen:
- EP-A- 0 712 831

## Beschreibung

Die vorliegende Erfindung betrifft ein verbessertes Verfahren zur Herstellung von 2-Fluor-1-cyclopropancarbonsäurederivaten durch Umsetzung von 2-Halogen-2-fluor-1-cyclopropancarbonsäurederivaten unter Zusatz eines Metalls und einer Base.

2-Fluor-1-cyclopropancarbonsäurederivate sind Zwischenprodukte zur Herstellung von pharmazeutischen Wirkstoffen, insbesondere von Antiinfektiva aus der Reihe der Chinolone (siehe z.B. Antimicrobial Agents and Chemotherapy 37 (12), 2747 bis 2753 (1993) und 38 (3), 611 bis 615 (1994) und J. Med. Chem. 37 (20), 3344 bis 3352 (1994)).

Der reduktive Austausch von Halogen in geminalen Halogen-Fluorcyclopropan-derivaten durch Wasserstoff ist in der Literatur schon beschrieben worden

Eine Umsetzung ist die Methode mit Tri-n-butyl-zinnhydrid (z.B. J. Am Chem Soc. 89, 5719 (1967), Tetrahedron Lett. 1967, 1123, J. Org. Chem. 35, 33 (1970) und Synthesis 1970, 499). Die Verwendung von Tri-n-butyl-zinnhydrid ist wegen seiner Toxizität, seiner schwierigen Zugänglichkeit, die hohe Kosten verursacht, und der benötigten großen Menge ökonomisch und ökologisch wenig sinnvoll.

Eine andere Methode ist die Umsetzung mit Natrium in flüssigem Ammoniak (s. z.B. Chem. Ber. 104, 1921 (1971)). Auch dieses Verfahren hat den Nachteil, daß es bei einer technischen Durchführung sehr aufwendig ist.

Bei einem weiteren Verfahren wird die Dehalogenierung mit anderen Metallen, bevorzugt Raney-Nickel, in Gegenwart von Wasserstoff und einer Base durchgeführt (s. z.B. J. Fluorine Chem. 49, 127 (1990) und WO 95/04712). In Bull Chem. Soc. Jap. 45, 1926 (1972) ist beschrieben, daß bei der hydrogenolytischen Dehalogenierung von 7-Chlor-7-fluorbicyclo[4.1.0]heptan mit Raney-Nickel nur mit 1,2-Diaminoethan als Base das gewünschte Produkt 7-Fluorbicyclo[4.1.0]heptan erhalten wurde und mit anderen Basen keine Reaktion stattfand.

Dieses Verfahren hat den Nachteil, daß die Selektivität gering ist. Man kann die Selektivität steigern, wenn man bei niedriger Temperatur (z.B. Raumtemperatur) arbeitet. Dies führt aber zu unvollständigem Umsatz und zu Reaktionszeiten von 50 Stunden und mehr. Auch bei Verwendung eines großen Überschußes an Raney-Nickel und Base kann der Umsatz nur wenig erhöht und die Reaktionszeit nur wenig verkürzt werden. So wird in der zuletzt zitierten Literatur beim Einsatz von 852 Mol-% Raney-Nickel und 6 000 Mol-% 1,2-Diaminoethan (jeweils bezogen auf das Edukt) in 24 Stunden bei 18 bis 22°C nur ein Umsatz von 58 % erreicht. Auch gemäß WO 95/04712 werden große Überschüsse an Raney-Nickel und 1,2-Diaminoethan eingesetzt, nämlich 466 bis 3785 Mol-% Raney-Nickel und 300 bis 1 000 Mol-% 1,2-Diaminoethan.

Es ist aber wesentlich, daß ein möglichst vollständiger Umsatz erreicht wird, da eine destillative Trennung der gewünschten Produkte, insbesondere der cis-Isomeren, von den Ausgangsprodukten mit vertretbarem Aufwand nicht möglich ist

Erhöht man die Reaktionstemperatur so verläuft die Reaktion zwar wesentlich schneller, wobei man auch die benötigten Mengen an Raney-Nickel und 1,2-Diaminoethan reduzieren kann, gleichzeitig geht aber die Selektivität stark zurück. So wird bei 80°C nur noch eine Ausbeute von 10 % d Th. erreicht (s. J. Fluorine Chem. 49, 127 (1990)).

Es besteht deshalb noch immer das Bedürfnis nach einem Verfahren, das in kurzer Reaktionszeit und bei geringen Einsatzmengen von Metallen und Basen das gewünschte Produkt in guten Ausbeuten und Selektivitäten liefert.

Es wurde nun ein Verfahren zur Herstellung von Cyclopropancarbonsäurederivaten der Formel 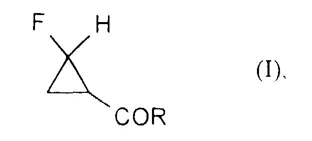 in der
- R: für OR¹ oder NR²R³ steht, wobei R¹, R² und R³ jeweils unabhängig voneinander für einen geradkettigen oder verzweigten C₁-C₄-Alkylrest stehen,
durch Umsetzung von Halogencyclopropancarbonsäurederivaten der Formel 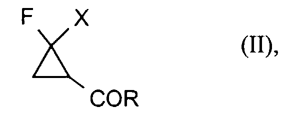 in der
- R: die bei Formel (I) angegebene Bedeutung hat und
- X: für Chlor, Brom oder Iod steht
in einem protischen Lösungsmittel, unter Zusatz eines Metalls und einer Base gefunden, das dadurch gekennzeichnet ist, daß man die Base während der Umsetzung zudosiert.

Man kann dann bei höheren Temperaturen, d.h. mit kürzeren Reaktionszeiten und verringertem Einsatz von Metallen und Basen arbeiten und trotzdem die Produkte der Formel (I) in guten Selektivitäten und Ausbeuten erhalten.

Mit C₁-C₄-Alkylresten sind insbesondere Methyl, Ethyl, Propyl, Isopropyl, Butyl, Isobutyl, s-Butyl und t-Butyl gemeint, bevorzugt Methyl und Ethyl, ganz besonders bevorzugt Ethyl.

In der Formel (I) steht X bevorzugt für Chlor und Brom, besonders bevorzugt für Chlor.

Bei dem erfindungsgemäß einzusetzenden Metall kann es sich beispielsweise um ein oder mehrere Metalle der 2, und/oder 8. Nebengruppe des Periodensystems der Elemente handeln, etwa um Zink, Eisen, Kobalt und/oder Nickel. Die Metalle können gegebenenfalls auf einen Träger aufgebracht sein oder in einer besonders aktiven Form vorliegen, z.B. in Raney-Form. Vorzugsweise werden Kobalt und/oder Nickel eingesetzt, besonders bevorzugt Nickel und ganz besonders bevorzugt Raney-Nickel.

Als Basen kommen für das erfindungnsgemäße Verfahren insbesondere organische Basen, beispielsweise Amine der Formel (III) in Frage 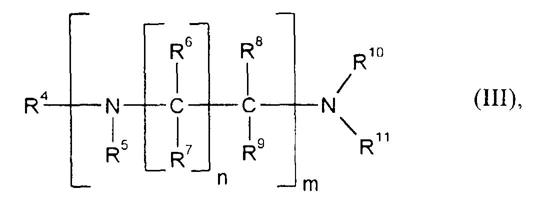 in der
- R⁴ bis R¹¹: jeweils unabhängig voneinander für Wasserstoff oder geradkettiges oder verzweigtes C₁-C₄-Alkyl,
- m: für 1, 2 oder 3 und
- n: für 1 oder 2 stehen.

Solche Amine sind beispielsweise 1,2-Ethylendiamin, Diethylentriamin, Triethylentetramin und Tetramethylethylendiamin Bevorzugt ist 1,2-Ethylendiamin. Man setzt i.a. nur eine Base ein, kann gegebenenfalls aber auch Gemische verschiedener Basen einsetzen.

Das erfindungsgemäße Verfahren wird in Gegenwart eines protischen Lösungsmittels durchgeführt. Als Lösungsmittel kommen z.B. Alkohole und Alkohole im Gemisch mit anderen Lösungsmitteln in Frage, z.B. Gemische mit Ethern und/oder aromatischen und/oder aliphatischen Kohlenwasserstoffen. Falls Ester als Ausgangsmaterial dienen (Formel (II), R = OR¹) ist es zweckmäßig als Lösungsmittel die in diesem Ester enthaltene Alkoholkomponente einzusetzen. Vorzugsweise werden wasserfreie Lösungsmittel verwendet oder solche, die weniger als 5 Gew.-% Wasser enthalten.

Man kann beispielsweise die 1- bis 6-fache, bevorzugt die 2- bis 4-fache Gewichtsmenge Lösungsmittel, bezogen auf die Verbindung der Formel (II), einsetzen.

Das erfindungsgemäße Verfahren kann man beispielsweise bei Temperaturen im Bereich von 0 bis 120°C, bevorzugt von 20 bis 100°C, besonders bevorzugt von 50 bis 80°C durchführen.

Es ist ein wesentliches Merkmal des erfindungsgemäßen Verfahrens, daß man die Base während der Umsetzung zudosiert. Beispielsweise kann man so verfahren, daß man das im Lösungsmittel gelöste Ausgangsmaterial und das Metall vorlegt, dieses Gemisch unter Rühren auf die gewünschten Reaktionsbedingungen bringt und dann im Verlaufe von z.B. 5 bis 20 Stunden, bevorzugt 10 bis 15 Stunden, die Base kontinuierlich in das Reaktionsgefäß einpumpt. Man kann die Base auch in kleinen Portionen, z.B. in Teilmengen von je 1 bis 10 Gew-% der Gesamtmenge, in das Reaktionsgemisch einbringen und nach jeder Zugabe, z.B. 3 Minuten bis 2 Stunden, unter Reaktionsbedingungen rühren. Bevorzugt ist die kontinuierliche Zugabe.

Man kann das erfindungsgemäße Verfahren mit oder ohne Zusatz von Wasserstoff durchführen Wenn man unter Zusatz von Wasserstoff arbeitet, kann der Druck in weiten Grenzen schwanken. Er kann beispielsweise 1,5 bis 200 bar, bevorzugt 5 bis 150 bar, insbesondere 10 bis 100 bar betragen.

Theoretisch benötigt man bei der Arbeitsweise unter Zusatz von Wasserstoff für den vollständigen Umsatz von 1 mol einer Verbindung der Formel (II) 1/2 mol Metall. Es ist deshalb zweckmäßig in diesem Fall, bezogen auf die Verbindung der Formel (II), mindestens 50 Mol-% eines Metalls einzusetzen. Bei der Arbeitsweise ohne Zusatz von Wasserstoff wird für den vollständigen Umsatz von 1 mol einer Verbindung der Formel (II) theoretisch 1 mol Metall benötigt. Deshalb ist es in diesem Fall zweckmäßig, bezogen auf die Verbindung der Formel (II) mindestens 100 mol.-% eines Metalls einzusetzen. Es ist vorteilhaft, das jeweilige Metall im Überschuß zu verwenden, jedoch bringen größere Mengen als 300 Mol-% Metall (bezogen auf die Verbindung der Formel (II)) i.a. keine Vorteile, sind aber wirtschaftlich nachteilig. Vorzugsweise setzt man 110 bis 250 Mol-%, insbesondere 120 bis 200 Mol-% Metall (bezogen auf die Verbindung der Formel (II)) ein, unabhängig davon, ob man mit oder ohne Zusatz von Wasserstoff arbeitet.

Theoretisch benötigt man bei der Arbeitsweise unter Zusatz von Wasserstoff für den vollständigen Umsatz von 1 mol einer Verbindung der Formel (II) 1,5 mole eines Amins der Formel (III). Es ist deshalb in diesem Fall zweckmäßig, bezogen auf die Verbindung der Formel (II) mindestens 150 Mol-% eines Amins der Formel (III) einzusetzen. Größere Mengen als 400 Mol-% eines Amins der Formel (III), bezogen auf die Verbindung der Formel (II), bringen in diesem Fall i.a. keine Vorteile, sind aber wirtschaftlich nachteilig. Vorzugsweise setzt man in diesem Fall 200 bis 300 Mol-%, insbesondere 220-270-Mol-% eines Amins der Formel (III), bezogen auf die Verbindung der Formel (II), ein. Andere Basen als Amine der Formel (III) kann man in entsprechenden Mengen einsetzen.

Bei der Arbeitsweise ohne Zusatz von Wasserstoff benötigt man für den vollständigen Umsatz von 1 mol einer Verbindung der Formel (II) theoretisch 3 mole eines Amins der Formel (III). Es ist deshalb in diesem Fall zweckmäßig, bezogen auf die Verbindung der Formel (II) mindestens 300 Mol-% eines Amins der Formel (III) einzusetzen. Hierbei bringen größere Mengen als 600 Mol-% eines Amins der Formel (III), bezogen auf die Verbindung der Formel (II), i.a. keine Vorteile, sind aber wirtschaftlich nachteilig. Vorzugsweise setzt man, wenn man ohne Zusatz von Wasserstoff arbeitet, 320 bis 500 Mol-%, insbesondere 350 bis 400 Mol-% eines Amins der Formel (III), bezogen auf die Verbindung der Formel (II), ein. Auch hier kann man andere Basen als Amine der Formel (III) in entsprechenden Mengen einsetzen.

Wenn man ohne Wasserstoffzusatz arbeitet kann man das erfindungsgemäße Verfahren beispielsweise in einem drucklos zu betreibenden Rührwerksbehälter durchführen. Die Arbeitsweise mit Wasserstoffzusatz erfordert druckfeste Gefäße, z.B. Autoklaven.

Die Aufarbeitung des ausreagierten Reaktionsgemisches kann z.B. so erfolgen, daß man zunächst vorliegende feste Bestandteile abtrennt, z.B. durch Filtration, dann zum Filtrat eine Säure, z.B. wäßrige Salzsäure, hinzufügt, um gegegebenenfalls noch vorhandene Base in das entsprechende Ammoniumsalz zu überführen. Danach kann man die gebildete organische Phase abtrennen, die wäßrige Phase mit einem geeigneten Lösungsmittel, z.B. Dichlormethan oder Toluol, extrahieren und schließlich die abgetrennte organische Phase und das Extrakt getrennt oder gemeinsam destillieren. Man kann die Säure dem Filtrat auch erst nach der Entfernung der Hauptmenge des darin enthaltenen Lösungsmittels zufügen.

Eine andere Aufarbeitungsmöglichkeit besteht darin, zunächst die vorhandenen Metallsalze durch Zusatz von Wasser zu lösen, dann das Metall abzutrennen, Säure zuzugeben und weiter wie oben beschrieben zu verfahren.

Neben den weiter oben geschilderten Vorteilen kann man mit dem erfindungsgemäßen Verfahren bei der Herstellung von 2-Fluorcyclopropancarbonsäureestern das häufig besonders erwünschte cis-Isomere in besonders guter Ausbeute und Selektivität erhalten.

Ob die Arbeitsweise mit oder ohne Wasserstoffzusatz vorteilhafter ist, kommt auf den Einzelfall an. Ohne Wasserstoffzusatz ist der apparative Aufwand geringer (keine druckfesten Apparate, keine Sicherheitsvorkehrungen für das Arbeiten mit Wasserstoff), jedoch ist dann die Effektivität des Verfahrens nicht optimal (höherer Chemikalienbedarf, nicht ganz so gute Ausbeuten und Selektivitäten, erhöhter Aufarbeitungs- und Entsorgungsaufwand). Die Arbeitsweise ohne Wasserstoffzusatz ist deshalb eher für die Herstellung geringerer Mengen von 2-Fluor-1-cyclopropancarbonsäurederivaten vorteilhaft.

Die Arbeitsweise mit Wasserstoffzusatz ist eher für die Herstellung größerer Mengen von 2-Fluorcyclopropancarbonsäurederivaten vorteilhaft, weil dann eine höhere Effektivität erzielt werden kann und die für diese Arbeitsweise erforderlichen höheren Investitionen schneller wieder erwirtschaftet werden können.

Es ist überraschend, daß sich die erfindungsgemäße Basendosierung in so starkem Maß vorteilhaft auswirkt (siehe das Vergleichsbeispiel) und erfindungsgemäß auch ohne Wasserstoffzusatz gearbeitet werden kann.

### Beispiele

In den folgenden Beispielen beziehen sich die Bezeichnungen "cis" und "trans" jeweils auf die Stellung des Fluoratoms zur Carboxylgruppe.

### Beispiel 1

In einem 3 l-Edelstahlautoklaven mit Ankerrührer wurden 270,0 g 2-Chlor-2-fluorcyclopropancarbonsäureethylester (Isomerenverhältnis cis:trans = 3:2), 1 350 ml Ethanol und 135 g mit Ethanol wasserfrei gewaschenes Raney-Nickel vorgelegt. Der Autoklav wurde mit Stickstoff gespült und unter Rühren auf 60°C aufgeheizt. Unter einem Wasserstoffdruck von 50 bar wurden 216,0 g 1,2-Ethylendiamin innerhalb von 15 Stunden kontinuierlich zugepumpt, was einer Zugabe von 14,4 g/h entsprach. Es wurde 16 Stunden nachgerührt Während der gesamten Reaktion und des Nachrührens wurden insgesamt 18 NL Wasserstoff aufgenommen.

Dann wurden die festen Bestandteile des Reaktionsgemischs abfiltriert und mit 500 ml Ethanol gewaschen. Das Filtrat und die Waschflüssigkeit wurden vereinigt und dann der größte Teil des Ethanols abdestilliert Das Destillat enthielt neben Ethanol nach GC-Analyse 35,6 g Trans-2-Fluorcyclopropancarbonsäureethylester (42,3 %, bezogen auf eingesetztes trans-Edukt), 4,6 g Cis-2-Fluorcyclopropancarbonsäureethylester (3,6 %, bezogen auf eingesetztes cis-Edukt) und 4,7 g Buttersäureethylester (2,5 %, bezogen auf die Summe von eingesetztem cis- und trans-Edukt).

Zum Destillationsrückstand wurden 250 ml Wasser gegeben und mit 37 gew.-%iger Salzsäure bei einer Temperatur von 22°C ein pH-Wert von 3 eingestellt. Die organische Phase, die sich im Verlauf der Salzsäurezugabe abzuscheiden begann, wurde abgetrennt, die wäßrige Phase dreimal mit jeweils 100 ml Methylenchlorid extrahiert, die organischen Phasen vereinigt und über Natriumsulfat getrocknet. Die vereinigten organischen Phasen enthielten nach GC-Analyse kein Ausgangsprodukt mehr.

Die Destillation der vereinigten organischen Phasen lieferte bei 20 mbar bei einer Sumpftemperatur von 35 bis 60°C und einer Kopftempertur von 38 bis 58°C ein Gemisch von 20,1 g Trans-2-Fluorcyclopropancarbonsäureethylester (23,9 %, bezogen auf eingesetztes trans-Edukt) 108,6 g Cis-2-Fluorcyclopropancarbonsäureethylester (85,9 %, bezogen auf eingesetztes cis-Edukt) und 0,5 g Buttersäureethylester (0,3 %, bezogen auf die Summe von eingesetztem cis- und trans-Edukt).

### Beispiel 2

In einem 3 l-Edelstahlautoklaven mit Ankerrührer wurden 270,0 g 2-Chlor-2-fluorcyclopropancarbonsäureethylester (Isomerenverhältnis cis:trans = 3:2), 1 350 ml Ethanol und 135 g mit Ethanol wasserfrei gewaschenes Raney-Nickel vorgelegt. Der Autoklav wurde mit Stickstoff gespült und unter Rühren auf 60°C aufgeheizt. Unter einem Wasserstoffdruck von 10 bar wurden 234,0 g Ethylendiamin innerhalb 11 Stunden kontinuierlich zugepumpt. Es wurde 14 Stunden nachgerührt. Insgesamt wurden ca. 18 NL Wasserstoff aufgenommen.

Zu der Reaktionslösung wurden bei 20°C 1 400 ml Wasser hinzugefügt. Die verbleibenden festen Bestandteile wurden abgesaugt und das Filtrat mit 30 gew.-%iger Salzsäure auf einen pH-Wert von 3 eingestellt. Es wurde sechsmal mit jeweils 100 ml Toluol extrahiert. Die organische Phase wurde mit gesättigter Natriumhydrogencarbonatlösung gewaschen. Die anschließende Destillation lieferte 48,9 g Trans-2-Fluorcyclopropancarbonsäureethylester (58,2 %, bezogen auf eingesetztes trans-Edukt), 99,9 g Cis-2-Fluorcyclopropancarbonsäureethylester (79,1 %, bezogen auf eingesetztes cis-Edukt) und 6,8 g Buttersäureethylester (3,7 %, bezogen auf die Summe von eingesetztem cis- und trans-Edukt)

### Beispiel 3

In einem 1 l-Dreihalskolben wurden 30,0 g 2-Chlor-2-fluorcyclopropancarbonsäureethylester (Isomerenverhältnis cis:trans = 3:2), 300 ml Ethanol und 30 g mit Ethanol wasserfrei gewaschenes Raney-Nickel vorgelegt. Der Kolbeninhalt wurde unter Rühren auf 80°C aufgeheizt und im Verlauf von 13 Stunden wurdem 33 g Ethylendiamin kontinuierlich zugepumpt. Es wurde noch 12 Stunden nachgerührt Nach dem Abkühlen auf Raumtemperatur wurden die festen Bestandteile des Reaktionsgemischs abgesaugt. Zum Filtrat wurden 100 ml Wasser hinzugefügt und mit 37 gew.-%iger wässriger Salzsäure ein pH-Wert von 3 eingestellt. Es wurde fünfmal mit jeweils 50 ml Toluol extrahiert und die vereinigten organischen Phasen mit Natriumsulfat getrocknet.

Die Destillation lieferte 3,8 g Trans-2-Fluorcyclopropancarbonsäureethylester (40,7 %, bezogen auf eingesetztes trans-Edukt), 9,1 g Cis-2-Fluorcyclopropancarbonsäureethylester (64,8 %, bezogen auf eingesetztes cis-Edukt) und 0,3 g Buttersäureethylester (1,5 %, bezogen auf die Summe von eingesetztem trans- und cis-Edukt).

### Vergleichsbeispiel

Es wurde verfahren wie in Beispiel 1, jedoch wurde das 1,2-Ethylendiamin nicht während der Reaktion kontinuierlich zugepumpt, sondern die Gesamtmenge zusammen mit den anderen Reaktionskomponenten im Autoklaven vorgelegt. Das abdestillierte Methanol enthielt nach GC-Analyse 22,9 g Trans-2-Flurocyclopropancarbonsäureethylester (27,2 %, bezogen auf eingesetztes trans-Edukt), 4,1 g Cis-2-Flurocyclopropancarbonsäureethylester (3,2 %, bezogen auf eingesetztes cis-Edukt) und 4,5 g Buttersäureethylester (2,4 %, bezogen auf die Summe von eingesetztem cis- und trans-Edukt). Die nach der Aufarbeitung vereinigten organischen Phasen enthielten kein Ausgangsprodukt mehr und ergaben bei der Destillation 12,1 g Trans-2-Flurocyclopropancarbonsäureethylester (14,4 %, bezogen auf eingesetztes trans-Edukt), 86,9 g Cis-2-Flurocyclopropancarbonsäureethylester (68,8 %, bezogen auf eingesetztes cis-Edukt) und 0,3 g Buttersäureethylester (0,2 %, bezogen auf die Summe von eingesetztem cis- und trans-Edukt).

Es ist ersichtlich, daß die erfindungsgemäße Arbeitsweise mit Zudosierung des Amins während der Reaktion hinsichtlich Selektivität und Ausbeute an Cis-2-Fluorcyclopropancarbonsäureethylester wesentlich bessere Ergebnisse liefert.

## Patentansprüche

1. Verfahren zur Herstellung von Cyclopropancarbonsäurederivaten der Formel 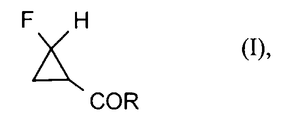 in der
R für OR¹ oder NR²R³ steht, wobei R¹, R² und R³ jeweils unabhängig voneinander für einen geradkettigen oder verzweigten C₁-C₄-Alkylrest stehen,
durch Umsetzung von Halogencyclopropancarbonsäurederivaten der Formel 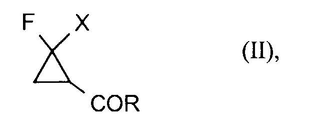 in der
R die bei Formel (I) angegebene Bedeutung hat und
X für Chlor, Brom oder Iod steht
in einem protischen Lösungsmittel, unter Zusatz eines Metalls und einer Base, dadurch gekennzeichnet, daß man die Base während der Umsetzung zudosiert.

2. Verfahren nach Anspruch 1, dadurch gekennzeichnet, daß man als Metall eines oder mehrere der 2. und/oder 8. Nebengruppe des Periodensystems der Elemente einsetzt.

3. Verfahren nach Ansprüchen 1 und 2, dadurch gekennzeichnet, daß man als Metall Raney-Nickel einsetzt.

4. Verfahren nach Ansprüchen 1 bis 3, dadurch gekennzeichnet, daß man als Base ein Amin der Formel 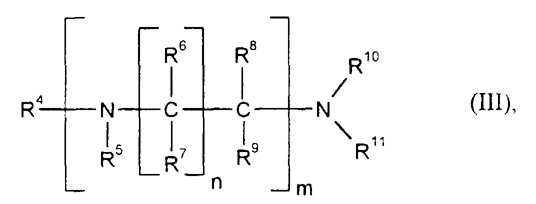 in der
R⁴ bis R¹¹ jeweils unabhängig voneinander für Wasserstoff oder geradkettiges oder verzweigtes C₁-C₄-Alkyl,
m für 1, 2 oder 3 und
n für 1 oder 2 stehen,
einsetzt.

5. Verfahren nach Ansprüchen 1 bis 4, dadurch gekennzeichnet, daß man als Base 1,2-Ethylendiamin einsetzt.

6. Verfahren nach Ansprüchen 1 bis 5, dadurch gekennzeichnet, daß man es bei Temperaturen im Bereich von 0 bis 120°C durchführt.

7. Verfahren nach Ansprüchen 1 bis 6, dadurch gekennzeichnet, daß man 110 bis 250 Mol-% Metall, bezogen auf die Verbindung der Formel (II), einsetzt.

8. Verfahren nach Ansprüchen 1 bis 7, dadurch gekennzeichnet, daß man unter Zusatz von Wasserstoff, bei Drucken im Bereich 1,5 bis 200 bar und unter Zusatz von 200 bis 300 Mol-% eines Amins der Formel (III), bezogen auf die Verbindung der Formel (II) arbeitet.

9. Verfahren nach Ansprüchen 1 bis 7, dadurch gekennzeichnet, daß man ohne Zusatz von Wasserstoff, drucklos und unter Zusatz von 320 bis 500 Mol-% eines Amins der Formel (III), bezogen auf die Verbindung der Formel (II) arbeitet.

10. Verfahren nach Ansprüchen 1 bis 9, dadurch gekennzeichnet, daß man die Base kontinuierlich in das Reaktionsgefäß einpumpt oder in kleinen Portionen von je 1 bis 10 Gew.-% der Gesamtmenge, in das Reaktionsgefäß einbringt und nach jeder Zugabe 3 Minuten bis 2 Stunden unter Reaktionsbedingungen rührt.

## Claims

1. Process for preparing cyclopropanecarboxylic acid derivatives of the formula 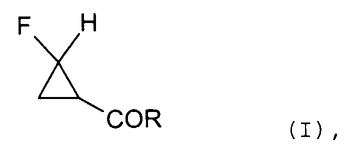 in which
R represents OR¹ or NR²R³, where R¹, R² and R³ independently of one another each represent a linear or branched C₁-C₄-alkyl radical,
by reacting halogenocyclopropanecarboxylic acid derivatives of the formula 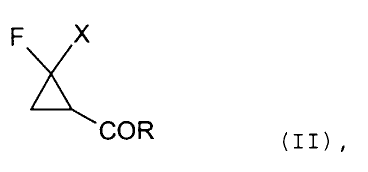 in which
R has the meaning specified under formula (I) and
X represents chlorine, bromine or iodine
in a protic solvent, with addition of a metal and a base, characterized in that the base is added during the reaction.

2. Process according to Claim 1, characterized in that as metal use is made of one or more of the elements of the 2nd and/or 8th subgroup of the Periodic Table of the Elements.

3. Process according to Claims 1 and 2, characterized in that the metal used is Raney nickel.

4. Process according to Claims 1 to 3, characterized in that the base used is an amine of the formula 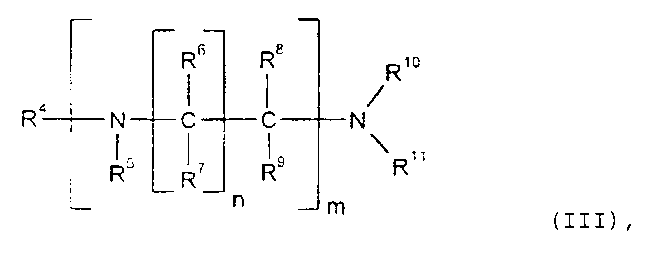 in which
R⁴ to R¹¹ independently of one another each represent hydrogen or linear or branched C₁-C₄-alkyl,
m represents 1, 2 or 3 and
n represents 1 or 2.

5. Process according to Claims 1 to 4, characterized in that the base used is 1,2-ethylenediamine.

6. Process according to Claims 1 to 5, characterized in that it is carried out at temperatures in the range from 0 to 120°C.

7. Process according to Claims 1 to 6, characterized in that 110 to 250 mol% of metal, based on the compound of the formula (II), are used.

8. Process according to Claims 1 to 7, characterized in that the procedure is carried out with addition of hydrogen, at pressures in the range 1.5 to 200 bar and with addition of 200 to 300 mol% of an amine of the formula (III), based on the compound of the formula (II).

9. Process according to Claims 1 to 7, characterized in that the procedure is carried out without addition of hydrogen, at atmospheric pressure and with addition of 320 to 500 mol% of an amine of the formula (III), based on the compound of the formula (II).

10. Process according to Claims 1 to 9, characterized in that the base is continuously pumped into the reaction vessel or is introduced into the reaction vessel in small portions each of 1 to 10% by weight of the total amount and, after each addition, the mixture is stirred under reaction conditions for 3 minutes to 2 hours.

## Revendications

1. Procédé pour la préparation de dérivés d'acides cyclopropanecarboxyliques répondant à la formule 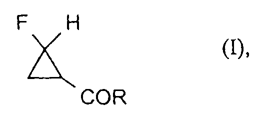 dans laquelle
R représente un groupe OR¹ ou un groupe NR²R³, dans lesquels R¹, R² et R³ représentent chacun indépendamment l'un de l'autre un radical alkyle en C₁-C₄ à chaîne droite ou ramifiée,
par mise en réaction de dérivés d'acides halogénocyclopropanecarboxyliques répondant à la formule 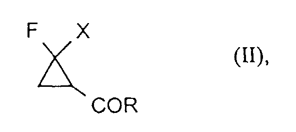 dans laquelle
R a la signification indiquée dans la formule (I), et
X représente un atome de chlore, un atome de brome ou un atome d'iode,
dans un solvant protique, avec addition d'un métal ou d'une base, caractérisé en ce qu'on ajoute la base de manière dosée au cours de la mise en réaction.

2. Procédé selon la revendication 1, caractérisé en ce qu'on met en oeuvre, à titre de métal, un ou plusieurs métaux choisis parmi le 2ème et/ou le 8ème sous-groupe du système périodique des éléments.

3. Procédé selon les revendications 1 et 2, caractérisé en ce qu'on met en oeuvre, à titre de métal, du nickel de Raney.

4. Procédé selon les revendications 1 à 3, caractérisé en ce qu'on met en oeuvre, à titre de base, une amine répondant à la formule 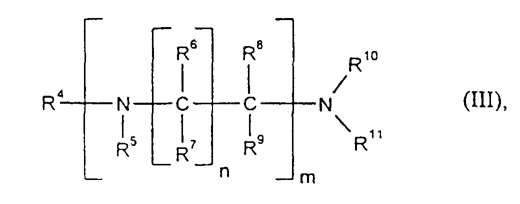 dans laquelle
R⁴ à R¹¹ représentent chacun indépendamment l'un de l'autre un atome d'hydrogène ou un radical alkyle en C₁-C₄ à chaîne droite ou ramifiée,
m représente 1, 2 ou 3, et
n représente 1 ou 2.

5. Procédé selon les revendications 1 à 4, caractérisé en ce qu'on met en oeuvre, à titre de base, la 1,2-éthylène-diamine.

6. Procédé selon les revendications 1 à 5, caractérisé en ce qu'on travaille à des températures entre 0 et 120°C.

7. Procédé selon les revendications 1 à 6, caractérisé en ce qu'on met en oeuvre, de 110 à 250 moles % de métal, rapportés au composé répondant à la formule (II).

8. Procédé selon les revendications 1 à 7, caractérisé en ce que, avec addition d'hydrogène, on travaille sous des pressions dans le domaine de 1,5 à 200 bar et avec addition de 200 à 300 moles % d'une amine répondant à la formule (III), rapportés au composé répondant à la formule (II).

9. Procédé selon les revendications 1 à 7, caractérisé en ce que, sans addition d'hydrogène, on travaille en l'absence de pression et avec addition de 320 à 500 moles % d'une amine répondant à la formule (III), rapportés au composé répondant à la formule (II).

10. Procédé selon les revendications 1 à 9, caractérisé en ce qu'on introduit la base en continu dans le récipient de réaction par pompage ou bien on l'introduit par petites portions de respectivement 1 à 10 % en poids de la quantité totale et, après chaque addition, on agite pendant un laps de temps de 3 minutes à 2 heures dans les conditions réactionnelles.
